Europäisches Patentamt

(19) European Patent Office

Office européen des brevets

(11) Numéro de publication: **0 181 672**
**B1**

(12) **FASCICULE DE BREVET EUROPÉEN**

(45) Date de publication du fascicule du brevet:
08.06.88

(21) Numéro de dépôt: **85201782.1**

(22) Date de dépôt: **04.11.85**

(51) Int. Cl.⁴: **E 21 B 43/24,** E 21 B 43/30,
E 21 B 43/18, E 21 C 43/00,
C 10 J 5/00

(54) **Procédé de production de méthane et d'hydrocarbures par hydrogénation souterraine du charbon.**

(30) Priorité: **14.11.84 BE 6048027**
**07.03.85 BE 214613**

(43) Date de publication de la demande:
**21.05.86 Bulletin 86/21**

(45) Mention de la délivrance du brevet:
**08.06.88 Bulletin 88/23**

(84) Etats contractants désignés:
**AT CH DE FR GB IT LI LU NL SE**

(56) Documents cité:
**BE-A-818 898**
**DE-A-2 342 790**
**DE-A-2 533 659**
**DE-B-1 231 638**
**US-A-3 933 447**
**US-A-3 973 628**

(73) Titulaire: **Institution pour le Développement de la**
**Gazéification Souterraine, rue du Chéra 200,**
**B-4000 Liège (BE)**

(72) Inventeur: **Ledent, Pierre, Parc de Sainval 5, B-4040**
**Tilff (BE)**

(74) Mandataire: **Dellicour, Paul, Office de Brevets E.**
**Dellicour rue Fabry 18/012, B-4000 Liège (BE)**

EP 0 181 672 B1

## Description

La présente invention concerne la production de méthane et d'hydrocarbures et est relative a un procédé de production par hydrogénation souterraine du charbon (voir p.e. DE-A-2 533 659).

Il est connu que la quantité de matières volatiles produite par la pyrolyse du charbon peut être largement augmentée, lorsque l'opération de pyrolyse est réalisée sous pression en presence d'un gaz à haute teneur en hydrogène.

Il est connu, egalement, que le résidu de semi-coke peut être gazéifié, suivant la réaction :

$$C + 2\,H_2 = CH_4 + 87,5\,kJ/mole.$$

Cette réaction ne peut se dérouler que dans des conditions bien précises. En l'absence de catalyseurs la température doit dépasser 800°C, pour que la vitesse de réaction atteigne une valeur acceptable. De plus, la reaction étant limitée par un équilibre chimique, il est nécessaire de travailler à très haute pression pour que le rendement de conversion de l'hydrogène en méthane atteigne un niveau élevé. Dans la pratique industrielle l'opération est réalisée dans une gamme de températures de 800°C à 900°C et dans une gamme de pressions de 80 bar à 100 bar.

La gazéification souterraine du charbon par un agent gazéifiant à haute teneur en hydrogène ne peut être envisagée que dans des gisements situés au-delà de 800 mètres de profondeur. En effet, la pression des fluides présents dans un gisement vierge est généralement proche de la pression hydrostatique maximale et c'est à la profondeur de 800 mètres que cette pression hydrostatique atteint 80 bar. C'est donc à une profondeur égale ou supérieure à 800 mètres que des réactions d'hydrogénogazéification peuvent être réalisées à la pression de 80 bar, tout en évitant les fuites de gaz à travers les terrains de recouvrement.

Une seconde condition est nécessaire pour que l'hydrogénopyrolyse et l'hydrogénogazéification du charbon puissent être réalisées "in situ". La zone réactionnelle doit avoir un volume suffisamment grand pour compenser la faible vitesse de réaction entre l'hydrogène et le carbone et, à l'intérieur dc ce volume, il faut assurer une distribution très uniforme de l'agent gazéifiant pour maintenir les températures dans l'intervalle requis et pour éviter qu'une importante fraction du gisement reste en dehors des zones gazéifiées.

Aucun des procédés de gazéification souterraine, qui se sont développés jusqu'à présent, ne satisfait à cette seconde condition. En effet, tous ces procédés reposent sur la creation préalable d'un chenal qui relie les sondages d'injection aux sondages de production du gaz. Il en résulte que les réactions de gazéification ne se déroulent que le long des surfaces qui limitent ce chenal et qu'une fraction non négligeable du débit d'agent gazéifiant peut traverser le gazogène, sans entrer en contact avec le charbon.

Dans le procédé, qui fait l'objet de la présente invention, la circulation de l'agent gazéifiant, au sein du gisement, se fait par filtration à travers le charbon vierge, sans qu'aucun circuit d'écoulement privilégié ait été préalablement créé. Cette condition est réalisée en utilisant une pression d'injection supérieure à la pression minimale de fracturation du gisement, en désignant ainsi la contrainte qui préexiste dans une direction perpendiculaire au plan des fissures à travers lesquelles s'effectue l'écoulement du gaz.

Les données expérimentales accumulées au cours de trois années d'essais réalisés à Thulin (Belgique), dans le cadre du projet belgo-allemand de gazéification souterraine du charbon, ont démontré que, lorsque l'on injecte un gaz et lorsque sa pression d'injection est augmentée progressivement, le dépassement de la pression minimale de fracturation ne conduit pas à la fracturation du charbon mais qu'elle provoque l'ouverture des très nombreuses fissures qui préexistent dans la masse du charbon, sans que l'ouverture moyenne de ces fissures dépasse cinq microns à dix microns. Dans ces conditions, il devient possible d'injecter dans la veine des débits gazeux très importants et de leur assurer une parfaite dispersion à travers le gisement.

Dans le procédé conforme à l'invention les puits de production du gaz sont maintenus à une pression égale ou supérieure à 80 bar et l'opération d'hydrogenogazéification se développe à partir de ces puits, le front de réaction se déplaçant à contre-courant du sens d'écoulement des gaz en laissant derrière lui une zone de vide qui se comble par foudroyage du toit, tout en conservant une très grande perméabilité.

Du fait de cette progression à contre-courant du front de gazéification, le gaz à haute teneur en hydrogène, utilisé comme agent gazéifiant, traverse tout d'abord une zone de charbon vierge, dont la température augmente progressivement, par transfert de chaleur en provenance de la zone de gazéification. Dans cette zone imprégnée d'hydrogène et dont la température va en augmentant, les réactions d'hydrogénopyrolyse se développent dans des conditions optimales. Les produits de cette hydrogénopyrolyse passent ensuite à travers la zone d'hydrogénogazéification, ce qui provoque le cracking des goudrons et des composés chimiques les plus denses, tout en permettant la subsistance d'hydrocarbures aromatiques de la famille du benzène.

L'operation d'hydrogénogazéification est amorcée par une opération préalable de combustion du charbon réalisée autour de chacun des puits de production du gaz, en alternant des periodes d'injection d'air et des périodes d'évacuation des gaz de combustion. Pour la réalisation de cette opération préliminaire la mise à feu peut être effectuée, à volonté, par préchauffage de l'air, par l'injection d'un produit chimique capable de s'enflammer spontanément au contact de l'air (par exemple le triéthylborane) ou par l'injection d'un agent oxydant (par exemple l'eau oxygénée à forte concentration ou l'air enrichi d'oxygène).

Si la chaleur dégagée par la réaction d'hydrogénogazéification est insuffisante pour que la température de la zone, de réaction se maintienne au niveau optimum, le réglage de la température de la zone de reaction est obtenu par l'addition contrôlée d'une faible proportion d'oxygène, dans le gaz à haute teneur en hydrogène

2

utilisé comme agent gazéifiant, la teneur en oxygène du mélange restant en déça de la limite d'inflammabilité.

Lorsque ce mélange d'agents gazéifiants parvient dans la zone de réaction, l'oxygène qu'il contient réagit avec le charbon ou avec l'hydrogène, suivant les réactions :

$$O_2 + C = CO_2 + 406 \text{ kJ/mole } O_2$$

et

$$O_2 + 2 H_2 = 2 H_2O + 482 \text{ kJ/mole } O_2$$

Il en résulte que l'introduction d'un pourcent d'oxygène dans le gaz utilisé comme agent gazéifiant a le même effet qu'un préchauffage de l'ordre de 130°C à 150°C.

Le procédé conforme à l'invention est illustré à titre d'exemples seulement par les dessins schématiques annexés dans lesquels sont représentées en :

Figure 1 une coupe dans le plan d'une couche de charbon à faible pendage, située en gisement vierge, à 1000 m de profondeur ;

Figure 2 une coupe verticale, suivant la direction XY de la figure 1 à l'endroit où la couche est recoupée par un des sondages de production du gaz ;

Figures 3 et 4 une variante de réalisation respectivement dans une coupe verticale et dans une vue en plan.

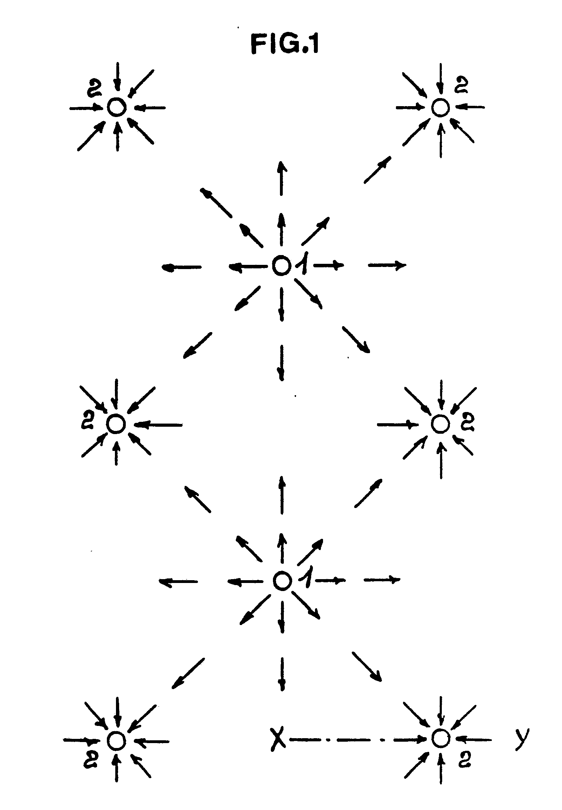

Le panneau à exploiter s'étend sur une surface de quelques hectares. Il est recoupé par huit sondages forés à partir de la surface. Les sondages 1, situés dans l'axe médian du panneau, secrvent à l'injection du gaz à haute teneur en hydrogène, utilisé comme agent gazéifiant. Les sondages 2, disposés à la périphérie du panneau, constituent les puits de production (figure 1).

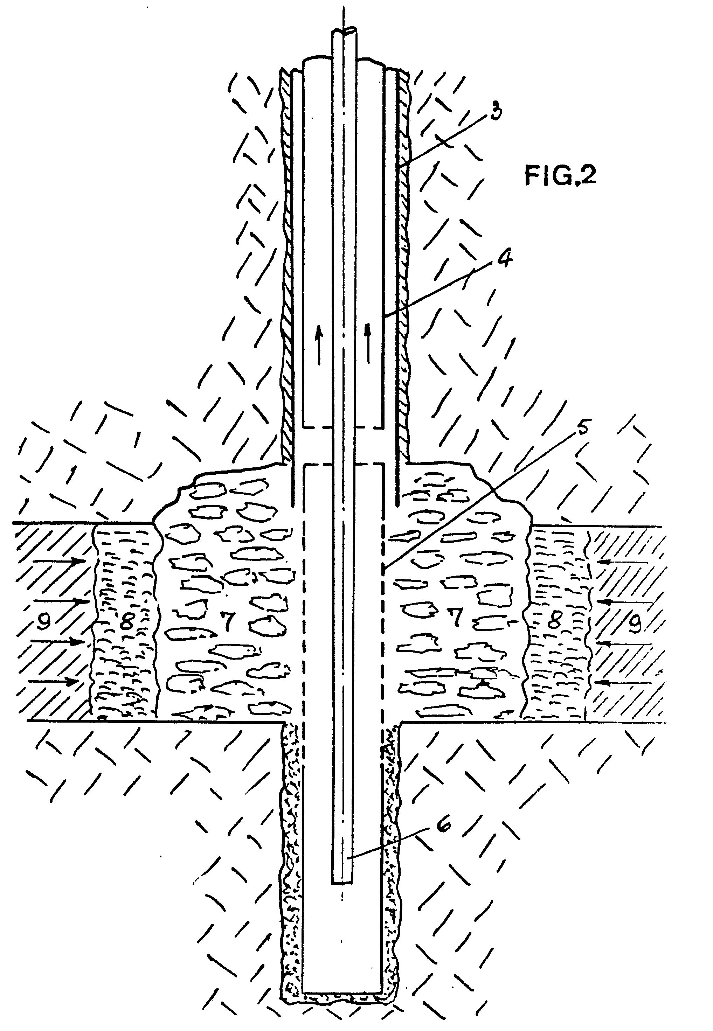

Comme le montre la figure 2 chacun de ces sondages est foré jusqu'à quelques mètres en dessous de la couche.

L'équipement de chaque sondage de production comporte :

- un casing métallique 3, qui se termine au toit de la couche et qui est relié par cimentation aux roches qui surmontent le gisement ;
- un tubing métallique 4, à travers lequel s'évacuent les gaz produits ;
- un liner 5 perforé au niveau de la veine pour permettre le passage du gaz tout en s'opposant au fluage du charbon ;
- un tubing 8, de petit diamètre, dont l'extrémité se situe en dessous du niveau de la couche et à travers lequel l'eau et les produits liquides, qui s'accumulent au fond du sondage, peuvent être évacués par transport pneumatique.

La mise en service de l'installation d'hydrogénogazéification débute par l'injection de gaz à haute teneur en hydrogène à travers les puits 1, sous une pression de l'ordre de 200 bar, et par le contrôle des débits récuperés par les puits de production périphériques 2 dans lesquels on maintient une contre-pression voisine de 100 bar.

On procède ensuite à l'allumage du charbon au fond des six sondages de production, en y injectant sous une pression de l'ordre de 200 bar un débit d'air prechauffé par passage sur un radiateur électrique ou un débit d'air suffisamment enrichi d'oxygène pour produire l'autoinflammation du charbon, sans nécessiter de préchauffage.

Pendant une période de quelques jours on entretient la combustion du charbon au fond des puits de production, en alternant des périodes d'injection d'air et des périodes de décompression des puits jusqu'à 100 bar.

Les injections d'air sont définitivement arrêtées, lorsque l'analyse des gaz récupérés fait apparaître une importante augmentation de la teneur en méthane, ce qui indique que les opérations d'hydrogénogazéification sont en cours.

A ce moment, la situation autour des puits de production 2 se présente suivant le schéma de la figure 2, où l'on peut distinguer trois zones concentriques successives autour de chacun des puits :

- une zone d'éboulis 7, qui correspond au vide produit par la combustion du charbon et comblé par le foisonnement du charbon de la zone suivante et par la chute de pierres provenant du toit de la couche ;
- une zone 8, constituée de charbon, qui a flué en direction du vide et qui, de ce fait, est devenu très perméable aux gaz ;
- une zone Y, constituée par le charbon vierge, qui est soumis à des pressions lithostatiques accrues et qui, en raison de sa très faible perméabilité, se comporte comme un diffuseur assurant une répartition très uniforme du gaz à haute teneur en hydrogène provenant des puits d'injection.

Les réactions d'hydrogénogazéification se développent dans la zone 8, qui offre un maximum de surfaces de contact entre gaz et solides. Simultanément, en raison de la conductibilité thermique du charbon, une onde de chaleur se propage de la zone 8 vers la zone 9, dans laquelle le chauffement progressif du charbon entraîne le développement de réactions d'hydrogénopyrolyse.

La consommation de charbon par les réactions d'hydrogénopyrolyse et d'hydrogénogazéification entraîne l'extension progressive des zones 7 et 8 et le déplacement du front de réaction à contre-courant du sens d'écoulement du gaz à haute teneur en hydrogène provenant des puits d'injection 1.

L'opération de gazéification se termine lorsque les fronts de réaction ont traversé tout le volume de charbon situé sur le parcours des gaz, qui s'écoulent des puits 1 vers les puits de production 2.

Le procédé conforme à l'invention est susceptible d'être développé suivant de nombreusos variantes.

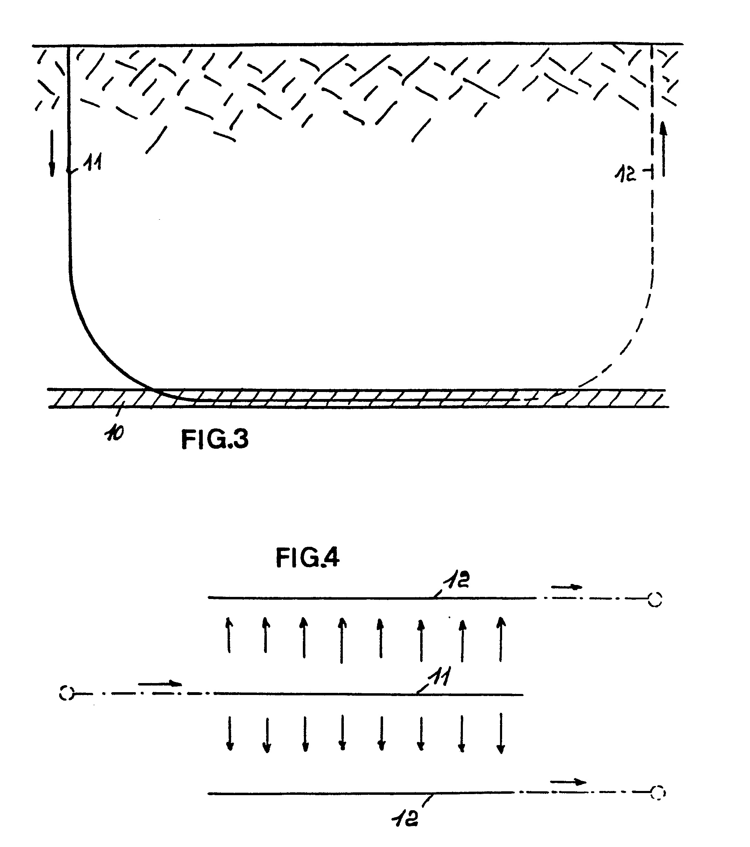

A titre d'exemple les figures 3 et 4 représentent une variante dans laquelle les sondages d'injection sont remplacés par un sondage unique 11, dévié jusque dans le plan de la couche 10 et dans laquelle les sondages de production sont remplacés par deux sondages déviés 12, forés aux limites du panneau à exploiter. La figure 3 représente une coupe verticale passant par le sondage 11 et la figure 4 une coupe dans le plan de la couche.

Pour illustrer l'intérêt économique du procédé conforme à l'invention, on trouvera, ci-dessous, une évaluation des résultats qui pourraient être obtenus dans un gazogène fonctionnant sous une pression de 100 bar et qui totaliserait 400 m² de fronts de réaction, dans une couche de 2 m d'ouverture.

| | |
|---|---|
| Vitesse de progression des fronts de réaction : | 0,05 m/h |
| Quantité de charbon consommé : | 400 x 0,05 x 1300 = 26 000 Kg/h |
| Température moyenne dans la zone de réaction : | 1100°K (827°C) |
| Composition du gaz produit : | $CH_4$ = 45 % $H_2$ = 36 % |
| | divers = 19 % |
| Débit d'hydrogène injecté : | 80 000 m³/h |
| Débit de gaz produit : | 70 000 m³/h |
| Chaleur potentielle de l'hydrogène injecté : | 1 020 GJ/h |
| Chaleur potentielle du charbon consommé : | 760 GJ/h |
| Chaleur potentielle du gaz et des sous-produits : | 1 638 GJ/h |
| Chaleur sensible du gaz et pertes de chaleur : | 142 GJ/h |
| Rendement de gazéification : | 81 % |

**Revendications**

1. Procédé de production de méthane et d'hydrocarbures par hydrogénation souterraine du charbon, dans un gisement vierge situé à grande profondeur recoupé par plusieurs sondages forés à partir de la surface, caractérisé en ce que le gaz, à haute teneur en hydrogène, utilisé comme agent gazéifiant est injecté, sous une pression supérieure à la pression minimale de fracturation du gisement, par un ou plusieurs sondages disposés dans l'axe du panneau à exploiter et en ce que les zones de réaction d'hydrogénation se développent à contrecourant du sens d'écoulement du gaz, sous une pression égale ou supérieure à 80 bar, à partir d'un certain nombre de sondages de production disposés à la péripherie du panneau à exploiter, la réaction étant amorcée par une opération préalable de combustion du charbon réalisés au fond de chacun des sondages de production.

2. Procédé de production de méthane et d'hydrocarbures suivant la revendication 1, caractérisé en ce que le réglage de la température de la zone de réaction est obtenu par l'addition contrôlée d'une faible proportion d'oxygène dans le gaz à haute teneur en hydrogène utilisé comme agent gazéifiant, la teneur en oxygène du mélange restant en deçà de la limite d'inflammabilité.

**Patentansprüche**

1. Verfahren zum Gewinnen von Methan und Kohlenwasserstoffen durch Untertage-Hydrierung von Kohle in einer in großer Tiefe liegenden sowie noch unberührten Lagerstätte, die durch mehrere von der Erdoberfläche ausgehenden Erdbohrungen erschlossen wird, dadurch gekennzeichnet, daß das als gasbildendes Mittel verwendete sowie einen hohen Wasserstoffgehalt aufweisende Gas unter einem Druck, der größer ist als der Minimaldruck, bei dem die Lagerstätte zusammenbricht, durch eine oder mehrere Erdbohrungen injiziert wird, die in der Achse des Arbeitsfeldes angeordnet sind und daß sich die Reaktionszonen der Hydrierung im Gegenstrom zur Strömungsrichtung des Gases unter einem Druck von größer oder gleich 80 bar ausgehend von einer vorgegebenen Anzahl von am Rand des Arbeitsfeldes angeordneten Erschließungsbohrungen entwickeln, wobei die Reaktion durch eine zuvor durchgeführte Verbrennung der Kohle am Grund jeder dieser Erschließungsbohrungen in Gang gesetzt worden ist.

2. Verfahren nach Anspruch 1, dadurch gekennzeichnet, daß das Regulieren der Temperatur in der Reaktionszone durch die kontrollierte Zugabe einer geringfügigen Menge an Sauerstoff an das als gasbildendes Mittel verwendete Gas mit dem hohen Wasserstoffgehalt erfolgt, wobei der Sauerstoffgehalt in dem Gemisch diesseits von der Entflammbarkeitsgrenze liegt.

**0 181 672**

**Claims**

1. Method of producing methane and hydrocarbons by subterranean hydrogenation of coal, in a virgin field situated at considerable depth and recovered via a plurality of bores drilled from the surface, characterised in that the gas, with a hydrogen content, used as a gasifying agent is in injected under a pressure in excess of the minimum fracturation pressure of the field via one or a plurality of bores disposed in the axis of the district to be worked and in that the hydrogenation reaction zones develop counter-current to the direction of gas flow, at a pressure equal to or greater than 80 bars, from a certain number of production bores disposed at the periphery of the district to be worked, the reaction being initiated by a prior operation to cause combustion of the coal, carried out at the bottom of the production bores.

2. Method of producing methane and hydrocarbons according to Claim 1, characterised in that the temperature at the reaction zone is regulated by the controlled addition of a small proportion of oxygen into the gas with a high hydrogen content, the hydrogen being used as a gasifying agent, the oxygen content of the mixture remaining within the limit of inflammability.

# FIG.1

0 181 672

FIG.2

FIG.3

FIG.4